# FASCICULE DE BREVET EUROPEEN

(11) **EP 1 745 771 B1**
(45) Date de publication et mention de la délivrance du brevet: **24.08.2011**
(21) Numéro de dépôt: 06116701.1
(22) Date de dépôt: 06.07.2006
(51) Int. Cl.: A61K 8/31, A61K 8/34, A61K 8/58, A61Q 1/10

(54) **Procédé de revêtement des cils**
Verfahren zur Beschichtung von Wimpern
Process for coating eyelashes

(30) Priorité: 22.07.2005 FR 0552287
(43) Date de publication de la demande: 24.01.2007
(73) Titulaire: L'Oréal, 75008 Paris (FR)
(72) Inventeur: Jager Lezer, Nathalie, 91370, Verrieres-Le-Buisson (FR); Lahousse, Florence, 94320, Thiais (FR)
(74) Mandataire: Duvert, Sandra

(56) Documents cités:
- EP-A- 0 374 332
- EP-A- 0 979 643
- EP-A- 1 249 224
- EP-A- 1 477 153
- EP-A- 1 600 146
- WO-A-2004/055079
- FR-A- 2 659 011
- FR-A- 2 863 493

## Description

La présente invention se rapporte à un procédé de revêtement des cils consistant à appliquer sur les fibres kératiniques une composition particulière selon la revendication 1.

La composition se présente sous la forme d'un mascara.
Par mascara, on entend une composition destinée à être appliquée sur les cils : il peut s'agir d'une composition de maquillage des cils, une base de maquillage des cils (aussi appelée base coat), une composition à appliquer sur un mascara, dite encore top-coat, ou bien encore une composition de traitement cosmétique des cils. Le mascara est plus particulièrement destiné aux cils d'êtres humains, mais également aux faux-cils.

D'une manière générale, les compositions de maquillage des cils ou mascaras sont constituées d'au moins une cire ou d'un mélange de cires dispersé dans une phase liquide aqueuse ou solvant organique. Ils présentent en général une texture pâteuse et sont conditionnés dans un récipient comprenant un réservoir muni d'un essoreur et d'un applicateur, notamment sous forme d'une brosse ou d'un peigne, et qui s'appliquent par prélèvement du produit dans le réservoir à l'aide de l'applicateur, passage de l'applicateur au travers de l'essoreur afin d'évacuer la surplus de produit, puis mise en contact de l'applicateur imprégné de mascara sur les cils.

Il est également connu par exemple des documents US 2 007 245 ou FR 2 833 163 des mascaras sous forme solide dits aussi « mascaras pains » qui sont des compositions comportant une forte proportion de cires, de pigments et de tensioactifs, délitables à l'eau c'est à dire qu'ils nécessitent, préalablement à leur application sur les cils, la mise en contact avec une phase aqueuse de manière à solubiliser partiellement le pain de mascara. En particulier l'application se fait via une brosse imprégnée d'eau qui est mise en contact avec le mascara puis le mélange prélevé et ensuite appliqué sur les cils avec la brosse de manière à déposer de la matière sur les cils. Le document US-A-222 896 décrit des mascaras solides applicables sans contact préalable avec une phase aqueuse mais ne décrit pas les ingrédients des compositions.

Les documents EP 1 249 224, EP 1 477 153, WO2004/055079, FR 2 863 493, EP 0 979 643 et FR 2 659 011 décrivent des compositions de maquillage, notamment des lèvres.

La présente invention a pour but de proposer une autre voie de formulation pour une composition de revêtement des fibres kératiniques, notamment des cils, qui permet en particulier une application rapide, directe et pratique sur les cils, sans l'intermédiaire d'une brosse (transfert de matière direct sur les cils) et applicable à sec.

Par « applicable à sec », on entend que la composition est apte à former un dépôt, de préférence adhérent et gainant, sur les fibres kératiniques sans nécessiter de mise en contact préalable avec une phase aqueuse, par opposition aux mascaras pains qui sont délitables à l'eau et doivent au préalable être solubilisés partiellement pour être appliqués sur les fibres et former un dépôt adhérent et gainant.

La composition mise en oeuvre dans le procédé selon l'invention présente en outre de bonnes propriétés de tenue (notamment à l'eau et à l'effritement) et un dépôt important et homogène de matière sur les cils.

De façon plus précise, l'invention a pour objet un procédé de revêtement des cils comprenant l'application sur les dites fibres kératiniques d'au moins une couche d'au moins une composition présentée sous forme d'un stick, ladite composition étant applicable à sec, selon la revendication 1.

Par stick, on désigne un bâton, de forme prédéterminée, généralement cylindrique, et qui, en l'absence de contrainte, à température ambiante et à pression atmosphérique, conserve sa forme prédéterminée. Ainsi conditionnée sous forme d'un stick, la composition est qualifiée d'auto-portée, de préférence pendant au moins 60 secondes. Généralement, de tels sticks sont obtenus par coulage à chaud de la composition dans un moule. Alternativement, de tels sticks peuvent être obtenus par extrusion.

La composition présente une dureté allant de 900 à 10000 Pa, et, de préférence, de 1800 à 8200 Pa.

Avec une telle dureté, la texture est assez « molle » pour permettre une application directe et aisée sur les cils, notamment un dépôt de matière par simple mise en contact avec les cils, sans exercer une pression exagérée sur la frange de cils.

Pour déterminer la dureté d'un stick conforme à l'invention, on peut utiliser la méthode dite « du fil à couper le beurre », qui consiste à couper transversalement le stick à l'aide d'un fil rigide de diamètre 250 µm en tungstène en faisant avancer le fil relativement au stick à une vitesse de 100 mm/min. La dureté correspond à la force maximale de cisaillement exercée par le fil sur le stick à 20 °C, cette force étant mesurée au moyen d'un dynamomètre DFGS2 commercialisé par la société INDELCO-CHATILLON. La mesure est reproduite 6 fois puis moyennée. La mesure est reproduite 6 fois. La moyenne des 6 valeurs lues au moyen du dynamomètre mentionné ci-dessus, notée Y, est donnée en grammes. Cette moyenne est convertie en Pascal par l'équation suivante pour obtenir la valeur de dureté :
(Y x 10⁻³ x 9,8) / surface de la section transversale du stick (en m²) Dans le cas d'un stick cylindrique de section circulaire, la surface de la section transversale est égale à TT x R², R étant le rayon du stick exprimé en mètres.

Selon cette méthode la dureté d'une composition cosmétique conforme au procédé de l'invention présentée sous la forme d'un stick varie, a plus ou moins 10% près, de 900 à 10000 Pa, et plus particulièrement de 1800 à 8200 Pa.

La composition utilisée dans le procédé selon l'invention comprend avantageusement une phase grasse liquide selon la revendication 1 et au moins un agent structurant selon la revendication 1 de ladite phase grasse liquide, via lesquels est ajustée la dureté de la composition.

### Phase grasse liquide

Par phase grasse liquide, au sens de la demande, on entend une phase grasse liquide à température ambiante (25°C) et pression atmosphérique (760 mm de Hg), composée d'un ou plusieurs corps gras non aqueux liquides à température ambiante, appelés aussi huiles ou, compatibles entre eux.
La phase grasse liquide comprend une huile volatile ou un mélange d'huiles volatiles selon la revendication 1.

La ou les huiles peuvent être présentes dans la composition en une teneur allant de 5 à 85 % en poids par rapport au poids total de la composition, de préférence de 10 à 70 % et de façon encore plus préférée de 15 à 60 % en poids.

Par " huile volatile", on entend au sens de l'invention une huile susceptible de s'évaporer au contact de la peau ou de la fibre kératinique en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 0,13 Pa à 40 000 Pa (10⁻³ à 300 mm de Hg), en particulier allant de 1,3 Pa à 13 000 Pa (0,01 à 100 mm de Hg), et plus particulièrement allant de de 1,3 Pa à 1300 Pa (0,01 à 10 mm de Hg).
Par "huile non volatile", on entend une huile restant sur la peau ou la fibre kératinique à température ambiante et pression atmosphérique au moins plusieurs heures et ayant notamment une pression de vapeur inférieure à 10⁻³ mm de Hg (0,13 Pa).
Ces huiles peuvent être des huiles hydrocarbonées, des huiles siliconées, des huiles fluorées, ou leurs mélanges.
On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C8-C16 comme les isoalcanes en C8-C16 d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en C8-C16 le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.
Comme huiles volatiles, on peut aussi utiliser les silicones volatiles, comme par exemple les huiles de silicones linéaires ou cycliques volatiles, notamment celles ayant une viscosité ≤ 8 centistokes (8 10⁻⁶ m²/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.
On peut également citer les huiles linéaires alkyltrisiloxanes volatiles de formule générale (I) 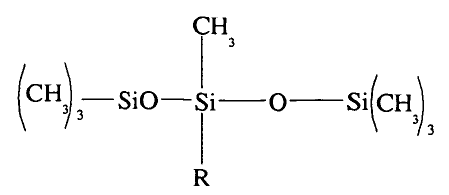 où R représente un groupe alkyle comprenant de 2 à 4 atomes de carbone et dont un ou plusieurs atomes d'hydrogène peuvent être substitués par un atome de fluor ou de chlore.
Parmi les huiles de formule générale (I), on peut citer :
le 3-butyl 1,1,1,3,5,5,5-heptaméthyl trisiloxane,
le 3-propyl 1,1,1,3,5,5,5-heptaméthyl trisiloxane, et
le 3-éthyl 1,1,1,3,5,5,5-heptaméthyl trisiloxane,
correspondant aux huiles de formule (I) pour lesquelles R est respectivement un groupe butyle, un groupe propyle ou un groupe éthyle.
On peut également utiliser des solvants volatils fluorés tels que le nonafluorométhoxybutane ou le perfluorométhylcyclopentane.

Selon l'invention, la composition mise en oeuvre comprend une huile volatile ou un mélange d'huiles volatiles, ou phase grasse liquide volatile, présentant dans la composition un profil d'évaporation tel que la masse d'huile(s) volatile(s) évaporée(s) au bout de trente minutes va de 1,7 à 370 mg/cm², notamment de 2 à 70 mg/cm², et en particulier de 2 à 30 mg/cm².
Le profil d'évaporation est mesuré selon le protocole suivant :
On introduit dans un cristallisoir (diamètre : 7 cm) placée sur une balance se trouvant dans une enceinte d'environ 0,3 m³ régulée en température (25 °C) et en hygrométrie (humidité relative 50 %) 15 g d'huile ou du mélange d'huiles à tester. On laisse le liquide s'évaporer librement, sans l'agiter, en assurant une ventilation par un ventilateur (PAPST -MOTOREN, référence 8550 N, tournant à 2700 tours/minute) disposé en position verticale au-dessus du cristallisoir contenant le solvant, les pales étant dirigées vers le cristallisoir et à une distance de 20 cm par rapport au fond du cristallisoir. On mesure à intervalles réguliers, et en particulier à trente minutes, la masse d'huile(s) restant dans le cristallisoir. Les vitesses d'évaporation sont exprimées en mg d'huile évaporée par unité de surface (cm²) et par unité de temps (minute).

De préférence encore, la composition comprend au moins une huile volatile choisie parmi les huiles volatiles hydrocarbonées ayant de 8 à 16 atomes de carbone, les huiles siliconées volatiles ayant de 2 à 7 atomes de silicium et leurs mélanges.

La composition peut également comprendre au moins une huile non volatile, et notamment choisie parmi les huiles hydrocarbonées et/ou siliconées et/ou fluorées non volatiles.
Comme huile hydrocarbonée non volatile, on peut notamment citer :
- les huiles hydrocarbonées d'origine végétale telles que les triesters d'acides gras et de glycérol dont les acides gras peuvent avoir des longueurs de chaînes variées de C4 à C24, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R1COOR2 dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que R1 + R2 soit ≥ 10, comme par exemple l'huile de Purcellin (octanoate de cétostéaryle), le myristate d'isopropyle, le palmitate d'isopropyle, le benzoate d'alcool en C12 à C15, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de diisostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
- les carbonates,
- les acétales,
- les citrates,
- et leurs mélanges.

Les huiles de silicone non volatiles utilisables dans la composition mise en oeuvre l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans l'invention sont notamment des huiles fluorosiliconées, des polyéthers fluorés, des silicones fluorées telles que décrit dans le document EP-A-847752.

Selon un mode de réalisation, la phase grasse peut comprendre une huile ester. Cette huile ester peut être choisie parmi les esters des acides monocarboxyliques avec les monoalcools et polyalcools.
Avantageusement, ledit ester répond à la formule (I) suivante :

R₁-CO-O-R₂ (I)

où R₁ représente un radical alkyle linéaire ou ramifié de 1 à 40 atomes de carbone, de préférence de 7 à 19 atomes de carbone, comprenant éventuellement une ou plusieurs double liaisons éthyléniques, et éventuellement substitué.
R₂ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, et éventuellement substitué.
Par « éventuellement substitué », on entend que R₁ et ou R₂ peuvent porter un ou plusieurs substituants choisis, par exemple, parmi les groupements comprenant un ou plusieurs hétéroatomes choisi parmi O, N et S, tels que amino, amine, alcoxy, hydroxyle.
De préférence, le nombre total d'atomes de carbone de R₁ + R₂ est ≥ 9.
R₁ peut représenter le reste d'un acide gras, de préférence supérieur, linéaire ou, de préférence ramifié comprenant de 1 à 40 et mieux de 7 à 19 atomes de carbone et R₂ peut représenter une chaîne hydrocarbonée linéaire ou de préférence ramifiée contenant de 1 à 40, de préférence de 3 à 30 et mieux de 3 à 20 atomes de carbone. De nouveau, de préférence, le nombre d'atomes de carbone de R₁ + R₂ ≥ 9.
Des exemples des groupes R₁ sont ceux dérivés des acides gras choisis dans le groupe constitué des acides acétique, propionique, butyrique, caproïque, caprylique, pélargonique, caprique, undécanoïque, laurique, myristique, palmitique, stéarique, isostéarique, arachidique, béhénique, oléique, linolénique, linoléïque, éléostéarique, arachidonique, érucique, et de leurs mélanges.
Des exemples d'esters sont, par exemple, l'huile de purcellin (octanoate de cétostéaryle), l'isononanoate d'isononyle, le myristate d'isopropyle, le palmitate d'éthyl-2-hexyle, le stéarate d'octyl 2-dodécyle, l'érucate d'octyl 2-dodécyle, l'isostéarate d'isostéaryle, et les heptanoates, octanoates, décanoates ou ricinoléates d'alcools ou de polyalcools, par exemple d'alcools gras.
Avantageusement, les esters sont choisis parmi les composés de la formule (I) ci-dessus, dans laquelle R₁ représente un groupe alkyle linéaire ou ramifié non substitué, comprenant éventuellement une ou plusieurs double liaisons éthyléniques, de 1 à 40 atomes de carbone, de préférence de 7 à 19 atomes de carbone, et R₂ représente un groupe alkyle linéaire ou ramifié non substitué, comprenant éventuellement une ou plusieurs double liaisons éthyléniques, de 1 à 40 atomes de carbone, de préférence de 3 à 30 atomes de carbone, et mieux de 3 à 20 atomes de carbone.
De préférence, R₁ est un groupe alkyle ramifié non substitué de 4 à 14 atomes de carbone, de préférence de 8 à 10 atomes de carbone et R₂ est un groupe alkyle ramifié non substitué de 5 à 15 atomes de carbone, de préférence de 9 à 11 atomes de carbone. De préférence dans la formule (I), R₁-CO- et R₂ ont le même nombre d'atomes de carbone et dérivent du même radical, de préférence alkyle ramifié non substitué, par exemple isononyle, c'est-à-dire que avantageusement la molécule d'huile ester est symétrique.
L'huile ester sera choisie, de préférence, parmi les composés suivants :
- l'isononanoate d'isononyle,
- l'octanoate de cétostéaryle,
- le myristate d'isopropyle,
- le palmitate d'éthyl-2-hexyle,
- le stéarate d'octyl 2-dodécyle,
- l'érucate d'octyl 2-dodécyle,
- l'isostéarate d'isostéaryle
- et leurs mélanges.

La phase grasse liquide peut représenter de 5 à 85% en poids par rapport au poids total de la composition, de préférence de 10 à 70% et de façon encore plus préférée de 15 à 60% en poids.

### Agent structurant

La composition mise en oeuvre comprend au moins un agent structurant de la phase grasse liquide (formée par les huiles ou solvants organiques volatiles ou non volatiles décrits plus haut) choisi parmi les cires.
L'agent structurant peut représenter de 1 à 50% en poids par rapport au poids total de la composition, de préférence de 5 à 20% et de façon encore plus préférée de 7,5 à 17% en poids
La quantité en structurant huileux peut être ajustée par l'homme du métier en fonction du propriétés de structuration desdits agents.

### Cire(s)

La cire considérée dans le cadre de la présente invention est d'une manière générale un composé lipophile, solide, déformable ou non déformable, à température ambiante (25 °C), à changement d'état solide/liquide réversible, ayant un point de fusion supérieur ou égal à 30 °C pouvant aller jusqu'à 200 °C et notamment jusqu'à 120 °C.
En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.
En particulier, les cires convenant à l'invention peuvent présenter un point de fusion supérieur ou égal à 45 °C, et en particulier supérieur ou égal à 55°C.
Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.
Le protocole de mesure est le suivant :
Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.
Les cires susceptibles d'être utilisées dans les compositions mises en oeuvre dans l'invention sont choisies parmi les cires, solides, à température ambiante d'origine animale, végétale, minérale ou de synthèse et leurs mélanges.
Les cires pouvant être utilisées dans les compositions mises en oeuvre dans l'invention présentent généralement une dureté allant de 0,5 MPa à 15 MPa, notamment supérieure à 1 MPa.
La dureté est déterminée par la mesure de la force en compression mesurée à 20 °C à l'aide du texturomètre vendu sous la dénomination TA-XT2 par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

### Le protocole de mesure est le suivant :

La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure de la dureté ou du collant.
Le mobile du texturomètre est déplacé à la vitesse de 0,1 mm/s, puis pénètre dans la cire jusqu'à une profondeur de pénétration de 0,3 mm. Lorsque le mobile a pénétré dans la cire à la profondeur de 0,3 mm, le mobile est maintenu fixe pendant 1 seconde (correspondant au temps de relaxation) puis est retiré à la vitesse de 0,5 mm/s.
La valeur de la dureté est la force de compression maximale mesurée divisée par la surface du cylindre du texturomètre en contact avec la cire.

La composition comprend au moins une cire choisie parmi :
- les cires dites « structurante », ayant peu d'affinité avec la phase grasse liquide de la composition, et au moins une cire choisie parmi
- les cires dites « non structurantes » présentant une affinité avec la phase grasse liquide de la composition
- et leurs mélanges.

Le caractère " structurant" ou "non structurant" de la cire est défini à partir de la valeur de dureté obtenue sur un mélange binaire constitué de 15% cire et 85% de la ou des huiles de la phase grasse liquide de la composition.
La dureté du mélange binaire est mesurée selon le protocole suivant :
On fait fondre la cire à une température d'environ 10°C au dessus du point de fusion de la cire sous agitation au barreau aimanté puis, après fusion totale de la cire, la ou les huiles sont ajoutées. L'agitation au barreau aimanté est maintenue pendant 30 min.
Le mélange est coulé dans un moule aluminium préalablement chauffé à 42°C, on laisse reposer 10 min à 25°C et l'ensemble est placé 20 minutes à -28°C, puis démoulé et conditionné dans un conditionnement de 12,3 mm de diamètre qui est conservé à une température de 20 °C, 24 heures avant de faire la mesure.
La dureté est mesurée par la méthode dite « du fil à couper le beurre », qui consiste à couper transversalement le stick à l'aide d'un fil rigide de diamètre 250 µm en tungstène en faisant avancer le fil relativement au stick à une vitesse de 100 mm/min. La dureté correspond à la force maximale de cisaillement exercée par le fil sur le stick à 20 °C, cette force étant mesurée au moyen d'un dynamomètre DFGS2 commercialisé par la société INDELCO-CHATILLON. La mesure est reproduite 6 fois puis moyennée. La dureté est exprimée en grammes.

Est considérée comme structurante une cire dont le mélange binaire tel que défini ci-dessus présente une dureté supérieure ou égale à 35 g (± 2 g).
Par opposition, est considérée comme non structurante une cire dont le mélange binaire tel que défini ci-dessus présente une dureté inférieure à 35 g (± 2 g).

On peut moduler la dureté de la composition en choisissant judicieusement les cires structurante et non structurante en fonction de la dureté du mélange binaire cire(s)-huile(s).

La composition comprend au moins un cire structurante et au moins un cire non structurante, qui sont présentes en un ratio cire structurante / cire non structurante allant de 15/85 à 35/65.

Les cires structurantes et/ou non structurantes peuvent être choisies parmi les cires protiques , les cires aprotiques et leurs mélanges.

### Cire aprotique

On entend par "cire aprotique", une cire comprenant peu ou ne comprenant pas d'atome d'hydrogène lié à un atome fortement électronégatif tel que O ou N.
De préférence, les cires aprotiques sont choisies parmi les cires apolaires, c'est-à-dire des cires constituées uniquement de molécules ne comportant que des atomes de carbone et d'hydrogène dans leur structure chimique, autrement dit ne comprenant pas d'hétéroatomes (tels que O, N, P) .

On peut citer à titre d'exemples de cires aprotiques, en particulier apolaires, les cires de paraffine, les cires microcristallines, l'ozokérite, la cérésine et les cires synthétiques comme les cires de polyméthylène, de polyéthylène, de propylène et leurs copolymères éthylène/propylène ou encore les cires de Fischer-Tropsch et leurs mélanges.
Peuvent être considérées comme aprotiques au sens de la présente invention, les cires obtenues par esterification ou modifiées par esterification et qui peuvent comprendre des groupes OH résiduels en fonction du rendement de l'esterification. De telles cires sont par exemple la cire issue de la réaction d'un acide gras sur un polyol ramifié de type ditrimethylol comme par exemple celles commercialisées sous la dénomination HEST par la société Heterene. On peut également citer les cires modifiées siliconées comme la cire de candellila siliconée commercialisée par Koster Keunen sous la dénomination Siliconyl candellila.
Sont également considérées comme aprotiques les cires obtenues par hydrogénation catalytique d'huiles animales ou végétales ayant des chaînes grasses, linéaires ou ramifiées, en C8-C32 telles que l'huile de jojoba hydrogénée, l'huile de tournesol hydrogénée, l'huile de coprah hydrogénée ou encore la cire obtenue par hydrogénation d'huile d'olive estérifiée avec l'alcool stéarylique.
La cire aprotique est de préférence choisie parmi les cires microcristallines, les cires de paraffine, les cires de polyéthylène, en particulier la cire commercialisée sous la référence WAX AC 617 par la société Honeywell, et leurs mélanges.

La ou les cire(s) aprotique(s) peu(ven)t être présente(s) en une teneur allant de en poids par rapport au poids total de la composition.

Par opposition aux cires aprotiques, sont considérées comme protiques les cires hydrocarbonées comme la cire d'abeille, la cire de lanoline ; la cire d'orange, la cire de citron, la cire de son de riz, la cire de Carnauba, la cire de Candellila, la cire d'Ouricury, la cire du Japon, la cire de Berry, la cire de shellac et la cire de sumac ; la cire de Montan, l'huile de ricin hydrogénée, l'huile de lanoline hydrogénée, les cires issues de la réaction d'acides gras sur des carbohydrates, comme les disaccharides de type sucrose, telles que le polybéhénate de sucrose, commercialisé par Croda sous la dénomination Cromaderm B, les cires hydroxyesters comme par exemple la cire (hydroxystéaryloxy)stéarate d'alkyle en C₂₀-C₄₀ telles que celles vendues sous les dénominations « Kester Wax K 82 P^{®} » et « Kester Wax K 80 P^{®} » par la société KOSTER KEUNEN. On peut également citer les cires alcools gras choisies parmi les alcools gras saturés ou non, ramifiés ou non, comportant de 20 à 60 atomes de carbone ou des mélanges comprenant au moins 30% desdits alcools gras, par exemple avec du polyéthylène, comme par exemple la cire commercialisée sous la référence PERFORMACOL® 550 L par la société NEW PHASE TECHNOLOGIES

La cire protique est de préférence choisie parmi la cire d'abeille, les cires alcool gras comprenant de 20 à 60 atomes de carbone et leurs mélanges.

Selon un mode de réalisation avantageux, la composition mise en oeuvre dans l'invention comprend au moins une cire protique, de préférence polaire, et au moins une cire aprotique, de préférence apolaire, choisies parmi les cires citées ci-dessus.

Selon un mode de réalisation de l'invention, la composition comprend au moins une phase grasse liquide comprenant au moins une huile hydrocarbonée volatile choisie parmi les isoparaffines ayant de 8 à 16 atomes de carbones, et notamment l'isododécane, au moins une cire aprotique, de préférence polaire choisie parmi les cires de polyéthylène, qui est non structurante, et au moins une cire polaire choisie parmi les cires alcool gras qui est structurante.

Selon un autre mode de réalisation de l'invention, la composition comprend au moins une phase grasse liquide comprenant au moins une huile siliconée volatile choisie parmi les huiles de silicones cycliques volatiles, notamment celles ayant une viscosité ≤ 8 centistokes (8 10⁻⁶ m²/s), et notamment la cyclopentadiméthylsiloxane, au moins une cire aprotique, de préférence apolaire, choisie parmi la cire de polyéthylène, qui est structurante en raison de son affinité avec les huiles siliconées, et au moins une cire protique, de préférence polaire choisie parmi la cire d'abeille, qui est non structurante.

La ou les cire(s) structurante et non structurante peu(ven)t représenter de 1 à 30% en poids par rapport au poids total de la composition, de préférence de 5 à 20% et de façon encore plus préférée de 7,5 à 17% en poids

### Composé pâteux

La composition mise en oeuvre dans l'invention peut comprendre au moins un composé pâteux.

Par "pâteux" au sens de la présente invention, on entend un composé gras lipophile à changement d'état solide/liquide réversible et comportant à la température de 23°C une fraction liquide et une fraction solide.

Le composé pâteux a de préférence une dureté à 20°C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20°C au centre de 5 échantillons. Le cylindre est introduit dans chaque échantillon à une pré-vitesse de 1 mm/s puis à une vitesse de mesure de 0,1 mm/s, la profondeur de pénétration étant de 0,3 mm. La valeur relevée de la dureté est celle du pic maximum.

Ce composé pâteux est en outre, à la température de 23°C, sous la forme d'une fraction liquide et d'une fraction solide. En d'autres termes, la température de fusion commençante du composé pâteux est inférieure à 23°C. La fraction liquide du composé pâteux mesurée à 23°C représente de 23 à 97% en poids du composé. Cette fraction liquide à 23°C représente de préférence entre 40 et 85% en poids du composé.

La fraction liquide en poids du composé pâteux à 23°C est égale au rapport de l'enthalpie de fusion consommée à 23°C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée en J/g.

L'enthalpie de fusion consommée à 23°C est la quantité d'énergie absorbée par l'échantillon pour passer de l'état solide à l'état qu'il présente à 23°C constitué d'une fraction liquide et d'une fraction solide.

La fraction liquide du composé pâteux mesurée à 32°C représente de préférence de 40 à 100% en poids du composé, de préférence de 50 à 100%, de préférence 80 à 100%, de préférence encore de 90 à 100% en poids du composé. Lorsque la fraction liquide du composé pâteux mesurée à 32°C est égale à 100%, la température de la fin de la plage de fusion du composé pâteux est inférieure ou égale à 32°C.

La fraction liquide du composé pâteux mesurée à 32°C est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.

Le composé pâteux est de préférence choisi parmi les composés synthétiques et les composés d'origine végétale. Un composé pâteux peut être obtenu par synthèse à partir de produits de départ d'origine végétale.

Le composé pâteux est avantageusement choisi parmi
- la lanoline et ses dérivés
- les composés siliconés polymères ou non
- les composés fluorés polymères ou non
- les polymères vinyliques, notamment:
   - les homopolymères d'oléfines
   - les copolymères d'oléfines
   - les homopolymères et copolymères de diènes hydrogénés
   - les oligomères linéaires ou ramifiés, homo ou copolymères de (méth)acrylates d'alkyles ayant de préférence un groupement alkyle en C₈-C₃₀
   - les oligomères homo et copolymères d'esters vinyliques ayant des groupements alkyles en C₈-C₃₀
   - les oligomères homo et copolymères de vinyléthers ayant des groupements alkyles en C₈-C₃₀,
- les polyéthers liposolubles résultant de la polyéthérification entre un ou plusieurs diols en C2-C100, de préférence en C2-C50,
- les esters et les polyesters,
et leurs mélanges.

Le composé pâteux est de préférence polymère, notamment hydrocarboné.

Un composé pâteux siliconé et fluoré préféré est le polyméthyl trifluoropropryl méthylalkyl diméthylsiloxane, fabriqué sous la dénomination X22-1088 par SHIN ETSU.

Lorsque le composé pâteux est un polymère siliconé et/ou fluoré, la composition comprend avantageusement un agent compatibilisant tel que les esters à courte chaîne comme le néopentanoate d'isodécyle.

Parmi les polyéthers liposolubles, on préfère en particulier les copolymères d'éthylène-oxyde et/ou de propylène-oxyde avec des alkylènes-oxydes à longue chaîne en C6-C30, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne sont disposés en blocs ayant un poids moléculaire moyen de 1.000 à 10.000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par Akzo Nobel.

Parmi les esters, on préfère notamment
- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol
- les esters de phytostérol,
- les esters de pentaérythritol
- les esters formés à partir
   - d'au moins un alcool, l'un au moins des alcools étant un alcool de Guerbet et
   - d'un dimère diacide formé à partir d'au moins un acide gras insaturé,
   comme l'ester de dimère d'acides gras de tallol comprenant 36 atomes de carbone et d'un mélange i) d'alcools de Guerbet comprenant 32 atomes de carbone et ii) d'alcool béhénylique; l'ester de dimère d'acide linoléique et d'un mélange de deux alcools de Guerbet, le 2-tétradécyl-octadécanol (32 atomes de carbone) et le 2-hexadécyl-eicosanol (36 atomes de carbone).
- les polyesters non réticulés résultant de la polycondensation entre un acide dicarboxylique ou un polyacide carboxylique linéaire ou ramifié en C4-C50 et un diol ou un polyol en C2-C50,
- les polyesters qui résultent de l'estérification, par un acide polycarboxylique d'un ester d'acide hydroxy carboxylique aliphatique comme le Risocast DA-L et le Risocast DA-H commercialisés par la société japonaise KOKYU ALCOHOL KOGYO, qui sont des esters résultant de la réaction d'estérification de l'huile de ricin hydrogénée avec l'acide dilinoléïque ou l'acide isostéarique
- les esters aliphatiques d'ester résultant de l'estérification d'un ester d'acide hydroxycarboxylique aliphatique par un acide carboxylique aliphatique; (Salacos HCIS (V)-L commercialisé par la société Nishing Oil)
L'acide carboxylique aliphatique comprend de 4 à 30 et de préférence de 8 à 30 atomes de carbone. Il est de préférence choisi parmi l'acide héxanoïque, l'acide heptanoïque, l'acide octanoïque, l'acide éthyl-2 héxanoïque, l'acide nonanoïque, l'acide décanoïque, l'acide undécanoïque, acide dodécanoïque, l'acide tridécanoïque, l'acide tétradécanoïque, l'acide pentadécanoïque, l'acide héxadécanoïque, l'acide héxyldécanoïque, l'acide heptadécanoïque, l'acide octadécanoïque, l'acide isostéarique, l'acide nonadécanoïque, l'acide eicosanoïque, l'acide isoarachidique, l'acide octyldodécanoïque, l'acide henéicosanoïque, l'acide docosanoïque, et leurs mélanges.
L'acide carboxylique aliphatique est de préférence ramifié.
L'ester d'acide hydroxy carboxylique aliphatique est avantageusement issu d'un acide carboxylique aliphatique hydroxylé comportant de 2 à 40 atomes de carbone, de préférence de 10 à 34 atomes de carbone et mieux de 12 à 28 atomes de carbone, et de 1 à 20 groupes hydroxyle, de préférence de 1 à 10 groupes hydroxyle et mieux de 1 à 6 groupes hydroxyle. L'ester d'acide hydroxy carboxylique aliphatique est choisi parmi :
a) les esters partiels ou totaux d'acides monocarboxyliques aliphatiques mono hydroxylés linéaires, saturés ;
b) les esters partiels ou totaux d'acides monocarboxyliques aliphatiques mono hydroxylés insaturés ;
c) les esters partiels ou totaux de polyacides carboxyliques aliphatiques mono hydroxylés saturés ;
d) les esters partiels ou totaux de polyacides carboxyliques aliphatiques poly hydroxylés saturés ;
e) les esters partiels ou totaux de polyols aliphatiques en C₂ à C₁₆ ayant réagi avec un mono ou un poly acide carboxylique aliphatique mono ou poly hydroxylé,
et leurs mélanges.

Les esters aliphatiques d'ester sont avantageusement choisis parmi :
- l'ester résultant de la réaction d'estérification de l'huile de ricin hydrogénée avec l'acide isostéarique dans les proportions 1 pour 1 (1/1) ou monoisostéarate d'huile de ricin hydrogénée,
- l'ester résultant de la réaction d'estérification de l'huile de ricin hydrogénée avec l'acide isostéarique dans les proportions 1 pour 2 (1/2) ou le diisostéarate d'huile de ricin hydrogénée,
- l'ester résultant de la réaction d'estérification de l'huile de ricin hydrogénée avec l'acide isostéarique dans les proportions 1 pour 3 (1/3) ou triisostéarate d'huile de ricin hydrogénée,
- et leurs mélanges.

Le composé pâteux représente de préférence 0,5 à 85%, mieux 1 à 60%, mieux 2 à 30% et mieux encore 5 à 15% en poids de la composition.

### Phase aqueuse

La composition mise en oeuvre dans l'invention peut comprendre une phase aqueuse qui peut être constituée essentiellement d'eau ; elle peut également comprendre un mélange d'eau et de solvant miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, les aldéhydes en C₂-C₄ et leur mélanges.

Selon un mode de réalisation, la composition comprend une phase aqueuse en une teneur inférieure à 10%, de préférence inférieure à 5% en poids, et mieux inférieure à 2% en poids par rapport au poids total de la composition.

Selon une autre mode de réalisation, la composition comprend une phase aqueuse en une teneur allant de 5 % à 95 % en poids, par rapport au poids total de la composition, de préférence allant de 10 % à 80 % en poids, et préférentiellement allant de 15 % à 60 % en poids.

### Système émulsionnant

La composition mise en oeuvre dans l'invention peut contenir des agents tensioactifs émulsionnants présents notamment en une proportion allant de 0,01 à 30 % en poids par rapport au poids total de la composition, mieux de 1 à 15 % et mieux de 2 à 10 %.
Selon l'invention, on utilise généralement un émulsionnant choisi de manière appropriée pour l'obtention d'une émulsion huile-dans-eau. En particulier, on peut utiliser un émulsionnant possédant à 25 °C une balance HLB (hydrophile-lipophile balance) au sens de GRIFFIN, supérieure ou égale à 8.
La valeur HLB selon GRIFFIN est définie dans J. Soc. Cosm. Chem. 1954 (volume 5), pages 249-256.
Ces agents tensioactifs peuvent être choisis parmi des agents tensioactifs non ioniques, anioniques, cationiques, amphotériques et leurs associations. On peut se reporter au document « Encyclopedia of Chemical Technology, KIRK-OTHMER », volume 22, p. 333-432, 3ème édition, 1979, WILEY, pour la définition des propriétés et des fonctions (émulsionnant) des tensioactifs, en particulier p. 347-377 de cette référence, pour les tensioactifs anioniques, amphotériques et non ioniques.

### Gélifiant hydrophile

Lorsqu'elle comprend une phase aqueuse, la composition mise en oeuvre dans l'invention peut comprendre un gélifiant hydrophile.
Les gélifiants hydrophiles utilisables dans les compositions mises en oeuvre peuvent être choisi parmi :
les homo- ou copolymères d'acides acrylique ou méthacrylique ou leurs sels et leurs esters et en particulier les produits vendus sous les dénominations VERSICOL F^{®} ou VERSICOL K^{®} par la société ALLIED COLLOID, UTRAHOLD 8^{®} par la société CIBA-GEIGY, les acides polyacryliques de type SYNTHALEN K,
les copolymères d'acide acrylique et d'acrylamide vendus sous la forme de leur sel de sodium sous les dénominations RETEN^{®} par la société HERCULES, le polyméthacrylate de sodium vendu sous la dénomination DARVAN N°7^{®} par la société VANDERBILT, les sels de sodium d'acides polyhydroxycarboxyliques vendus sous la dénomination HYDAGEN F^{®} par la société HENKEL,
les copolymères acide polyacryliques/acrylates d'alkyle de type PEMULEN,
l'AMPS (Acide polyacrylamidométhyl propane sulfonique neutralisé partiellement à l'ammoniaque et hautement réticulé) commercialisé par la société CLARIANT,
les copolymères AMPS/acrylamide de type SEPIGEL^{®} ou SIMULGEL^{®} commercialisés par la société SEPPIC, et
les copolymères AMPS/méthacrylates d'alkyle polyoxyéthylénés (réticulés ou non), et leurs mélanges.
Les polymères filmogènes hydrosolubles cités plus bas peuvent également jouer le rôle de gélifiant hydrophile.
Le gélifiant hydrophile peut être présent dans la composition en une teneur en matières sèches allant de 0,01 % à 30 % en poids, de préférence de 0,5 % à 20 % en poids, mieux de 1 % à 15 % en poids par rapport au poids total de la composition.

### Polymère filmogène

La composition mise en oeuvre dans l'invention peut comprendre selon un mode de réalisation particulier au moins un polymère filmogène. Le polymère filmogène peut être présent dans la composition en une teneur en matières sèches (ou matières actives) allant de 0,1 % à 30 % en poids par rapport au poids total de la composition, de préférence de 0,5 % à 20 % en poids, et mieux de 1 % à 15 % en poids.
Dans la présente invention, on entend par « polymère filmogène », un polymère apte à former à lui seul ou en présence d'un agent auxiliaire de filmification, un film macroscopiquement continu et adhérent sur les cils, et de préférence un film cohésif, et mieux encore un film dont la cohésion et les propriétés mécaniques sont telles que ledit film peut être isolable et manipulable isolément, par exemple lorsque ledit film est réalisé par coulage sur une surface antiadhérente comme une surface téflonnée ou siliconnée.
Parmi les polymères filmogènes utilisables dans la composition on peut citer les polymères synthétiques, de type radicalaire ou de type polycondensat, les polymères d'origine naturelle, et leurs mélanges.
Par polymère filmogène radicalaire, on entend un polymère obtenu par polymérisation de monomères à insaturation notamment éthylénique, chaque monomère étant susceptible de s'homopolymériser (à l'inverse des polycondensats).
Les polymères filmogènes de type radicalaire peuvent être notamment des polymères, ou des copolymères, vinyliques, notamment des polymères acryliques.
Les polymères filmogènes vinyliques peuvent résulter de la polymérisation de monomères à insaturation éthylénique ayant au moins un groupement acide et/ou des esters de ces monomères acides et/ou des amides de ces monomères acides.
Comme monomère porteur de groupement acide, on peut utiliser des acides carboxyliques insaturés α, β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.
Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₃₀, de préférence en C₁-C₂₀, des (méth)acrylates d'aryle, en particulier d'aryle en C₆-C₁₀, des (méth)acrylates d'hydroxyalkyle, en particulier d'hydroxyalkyle en C₂-C₆.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.
Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.
Comme amides des monomères acides, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide, le N-t-octyl acrylamide et le N-undécylacrylamide.
Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.
Parmi les polycondensats filmogènes, on peut citer les polyuréthanes, les polyesters, les polyesters amides, les polyamides, et les résines époxyesters, les polyurées.
Les polyuréthanes peuvent être choisis parmi les polyuréthanes anioniques, cationiques, non-ioniques ou amphotères, les polyuréthanes-acryliques, les poly-uréthanes-polyvinylpirrolidones, les polyester-polyuréthanes, les polyéther-polyuréthanes, les polyurées, les polyurée-polyuréthanes, et leurs mélanges.
Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.
Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.
Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.
Le polyester peut en outre comprendre au moins un monomère portant au moins un groupement -SO₃M, avec M représentant un atome d'hydrogène, un ion ammonium NH₄⁺ ou un ion métallique, comme par exemple un ion Na⁺, Li⁺, K+, Mg²⁺, Ca²⁺, Cu²⁺, Fe²⁺, Fe³⁺. On peut utiliser notamment un monomère aromatique bifonctionnel comportant un tel groupement -SO₃M.
Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphtalène-2,7-dicarboxylique.
On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique.
Les polymères d'origine naturelle, éventuellement modifiés, peuvent être choisis parmi la résine shellac, la gomme de sandaraque, les dammars, les élémis, les copals, les polymères cellulosiques, et leurs mélanges.
Selon un premier mode de réalisation de la composition mise en oeuvre dans l'invention, le polymère filmogène peut être un polymère hydrosoluble et peut être présent dans une phase aqueuse de la composition ; le polymère est donc solubilisé dans la phase aqueuse de la composition.

Selon une autre variante de réalisation de la composition mise en oeuvre dans l'invention, le polymère filmogène peut être un polymère solubilisé dans une phase grasse liquide comprenant des huiles ou solvants organiques tels que ceux décrits précédemment (on dit alors que le polymère filmogène est un polymère liposoluble). De préférence, la phase grasse liquide comprend une huile volatile, éventuellement en mélange avec une huile non volatile, les huiles pouvant être choisies parmi les huiles citées précédemment.
A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).
Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.
Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.
Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.
De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.
Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.
Comme polymères filmogènes liposolubles utilisables dans l'invention, on peut également citer les polyalkylènes et notamment les copolymères d'alcènes en C2-C20, comme le polybutène, les alkylcelluloses avec un radical alkyle linéaire ou ramifié, saturé ou non en C1 à C8 comme l'éthylcellulose et la propylcellulose, les copolymères de la vinylpyrolidone (VP) et notamment les copolymères de la vinylpyrrolidone et d'alcène en C2 à C40 et mieux en C3 à C20. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.
On peut également citer les résines de silicone, généralement solubles ou gonflables dans les huiles de silicone, qui sont des polymères de polyorganosiloxanes réticulés. La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.
A titre d'exemples de résines polymethylsilsesquioxanes commercialement disponibles, on peut citer celles qui sont commercialisés :
par la société Wacker sous la référence Resin MK tels que la Belsil PMS MK :
par la société SHIN-ETSU sous les références KR-220L.
Comme résines siloxysilicates, on peut citer les résines trimethylsiloxysilicate (TMS) telles que celle commercialisées sous la référence SR1000 par la société General Electric ou sous la référence TMS 803 par la société Wacker. On peut encore citer les résines timéthylsiloxysilicate commercialisées dans un solvant tel que la cyclomethicone, vendues sous la dénomination "KF-7312J" par la société Shin-Etsu, "DC 749", "DC 593" par la société Dow Corning.
On peut aussi citer des copolymères de résines de silicone telles que celles citées ci-dessus avec des polydiméthylsiloxanes, comme les copolymères adhésifs sensibles à la pression commercialisés par la société Dow Corning sous la référence BIO-PSA et décrits dans le document US 5 162 410 ou encore les copolymères siliconés issus de la réaction d'un résine de silicone, telle que celles décrite plus haut, et d'un diorganosiloxane tels que décrits dans le document WO 2004/073626.

Selon un mode de réalisation de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.
Avantageusement, les première et deuxième séquences et du polymère séquencé sont incompatibles l'une avec l'autre.
De tels polymères sont décrits par exemple dans les documents EP 1411069 ou WO04/028488.

Le polymère filmogène peut être également présent dans la composition sous la forme de particules en dispersion dans une phase aqueuse ou dans une phase solvant non aqueuse, connue généralement sous le nom de latex ou pseudolatex. Les techniques de préparation de ces dispersions sont bien connues de l'homme du métier.
Comme dispersion aqueuse de polymère filmogène, on peut utiliser les dispersions acryliques vendues sous les dénominations Neocryl XK-90^{®}, Neocryl A-1070^{®}, Neocryl A-1090^{®}, Neocryl BT-62^{®}, Neocryl A-1079^{®} et Neocryl A-523^{®} par la société AVECIA-NEORESINS, Dow Latex 432^{®} par la société DOW CHEMICAL, Daitosol 5000 AD^{®} ou Daitosol 5000 SJ^{®} par la société DAITO KASEY KOGYO; Syntran 5760^{®} par la société Interpolymer, Allianz OPT par la société ROHM & HAAS, les dispersions aqueuses de polymères acryliques ou styrène/acrylique vendues sous le nom de marque JONCRYL^{®} par la société JOHNSON POLYMER ou encore les dispersions aqueuses de polyuréthane vendues sous les dénominations Neorez R-981^{®} et Neorez R-974^{®} par la société AVECIA-NEORESINS, les Avalure UR-405^{®}, Avalure UR-410^{®}, Avalure UR-425^{®}, Avalure UR-450^{®}, Sancure 875^{®}, Sancure 861^{®}, Sancure 878^{®} et Sancure 2060^{®} par la société GOODRICH, Impranil 85^{®} par la société BAYER, Aquamere H-1511^{®} par la société HYDROMER ; les sulfopolyesters vendus sous le nom de marque Eastman AQ^{®} par la société Eastman Chemical Products, les dispersions vinyliques comme le Mexomère PAM^{®} de la société CHIMEX et leurs mélanges.
Comme exemples de dispersions non aqueuses de polymère filmogène, on peut citer les dispersions acryliques dans l'isododécane comme le Mexomère PAP^{®} de la société CHIMEX, les dispersions de particules d'un polymère éthylénique greffé, de préférence acrylique, dans une phase grasse liquide, le polymère éthylénique étant avantageusement dispersé en l'absence de stabilisant additionnel en surface des particules telles que décrite notamment dans le document WO 04/055081.

La composition mise en oeuvre dans l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

### Matière colorante

La composition mise en oeuvre dans l'invention peut également comprendre au moins une matière colorante comme les matières pulvérulentes, les colorants liposolubles, les colorants hydrosolubles.
Les matières colorantes pulvérulentes peuvent être choisies parmi les pigments et les nacres.
Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.
Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.
Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.
Ces matières colorantes peuvent être présentes en une teneur allant de 0,01 à 30 % en poids par rapport au poids total de la composition.

### Charges

La composition mise en oeuvre dans l'invention peut en outre comprendre au moins une charge.
Les charges peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon^{®} commercialisé sous la dénomination Orgasol^{®} par la société Atochem, de poly-β-alanine et de polyéthylène, les poudres de polymères de tétrafluoroéthylène comme le Téflon^{®}, la lauroyl-lysine, l'amidon, le nitrure de bore, les micro sphères creuses polymériques expansées telles que celles de chlorure de polyvinylidène/acrylonitrile comme celles commercialisées sous la dénomination d'Expancel^{®} par la société Nobel Industrie, les poudres acryliques telles que celles commercialisées sous la dénomination Polytrap^{®} par la société Dow Corning, les particules de polyméthacrylate de méthyle et les microbilles de résine de silicone (Tospearls^{®} de Toshiba, par exemple), le carbonate de calcium précipité, le carbonate et l'hydro-carbonate de magnésium, l'hydroxyapatite, les microsphères de silice creuses (Silica Beads^{®} de MAPRECOS), les microcapsules de verre ou de céramique, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc, le myristate de magnésium.
On peut également utiliser un composé susceptibles de gonfler à la chaleur et notamment des particules thermoexpansibles telles que les microsphères non expansées de copolymère de chlorure de vinylidène/d'acrylonitrile/méthacrylate de méthyle ou de copolymère d'homopolymère d'acrylonitrile comme par exemple celles commercialisées respectivement sous les références Expancel^{®} 820 DU 40 et Expancel^{®}007WU par la Société AKZO NOBEL. Les charges peuvent représenter de 0,1 à 25 %, en particulier de 1 à 20 % en poids par rapport au poids total de la composition.

L'agent de coloration ou la charge peuvent en outre être présents sous forme de « pâte particulaire ».

La composition, lorsqu'elle contient des particules solides à température ambiante, est préparée en les introduisant dans la composition sous forme d'une dispersion colloïdale appelée aussi "pâte particulaire", telle que décrite dans la demande WO 02/39961 dont le contenu est incorporé par référence dans la présente demande.

Par dispersion colloïdale ou "pâte particulaire", on entend au sens de l'invention une dispersion colloïdale concentrée de particules enrobées ou non dans un milieu continu, stabilisée à l'aide d'un agent dispersant ou éventuellement sans agent dispersant. Ces particules peuvent être choisies parmi les pigments, les nacres, les charges solides et leurs mélanges. Ces particules peuvent être de toute forme notamment de forme sphérique ou allongée comme des fibres. Elles sont insolubles dans le milieu.

L'agent dispersant sert à protéger les particules dispersées contre leur agglomération ou floculation. La concentration en dispersant généralement utilisée pour stabiliser une dispersion colloïdale est de 0,3 à 5 mg/m², de préférence de 0,5 à 4 mg/m², de surface de particules. Cet agent dispersant peut être un tensioactif, un oligomère, un polymère ou un mélange de plusieurs d'entre eux, portant une ou des fonctionnalités ayant une affinité forte pour la surface des particules à disperser. En particulier, ils peuvent s'accrocher physiquement ou chimiquement à la surface des pigments. Ces dispersants présentent, en outre, au moins un groupe fonctionnel compatible ou soluble dans le milieu continu. En particulier, on utilise les esters de l'acide hydroxy-12 stéarique en particulier et d'acide gras en C₈ à C₂₀ et de polyol comme le glycérol, la diglycérine, tel que le stéarate d'acide poly(12-hydroxystéarique) de poids moléculaire d'environ 750g/mole tel que celui vendu sous le nom de Solsperse 21 000 par la société Avecia, le polygycéryl-2 dipolyhydroxystéarate (nom CTFA) vendu sous la référence Dehymyls PGPH par la société Henkel ou encore l'acide polyhydroxystéarique tel que celui vendu sous la référence Arlacel P100 par la société Uniqema et leurs mélanges.

Comme autre dispersant utilisable dans la composition, on peut citer les dérivés ammonium quaternaire d'acides gras polycondensés comme le Solsperse 17 000 vendu par la société Avecia, les mélanges de poly diméthylsiloxane/oxypropylène tels que ceux vendus par la société Dow Corning sous les références DC2-5185, DC2-5225 C.

L'acide polydihydroxystéarique et les esters de l'acide hydroxy-12-stéarique sont de préférence destinés à un milieu hydrocarboné ou fluoré, alors que les mélanges de diméthylsiloxane oxyéthylène/oxypropyléné sont de préférence destinés à un milieu siliconé.

La dispersion colloïdale est une suspension de particules de taille généralement micronique (<10 µm) dans un milieu continu. La fraction volumique de particules dans une dispersion concentrée est de 20 % à 40 %, de préférence supérieure à 30 %, ce qui correspond à une teneur pondérale pouvant aller jusqu'à 70 % selon la densité des particules.

Les particules dispersées dans le milieu peuvent être constituées de particules minérales ou organiques ou de leurs mélanges tels que ceux décrits ci-après.

Le milieu continu de la pâte peut être quelconque et contenir tout solvant ou corps gras liquide et leurs mélanges. Avantageusement, le milieu liquide de la pâte particulaire est l'un des corps gras liquides ou huiles que l'on souhaite utiliser dans la composition, faisant ainsi partie de la phase grasse liquide.

De façon avantageuse, la "pâte particulaire" ou dispersion colloïdale est une "pâte pigmentaire" contenant une dispersion colloïdale de particules colorées, enrobées ou non. Ces particules colorées sont des pigments, des nacres ou un mélange de pigments et/ou de nacres.

Avantageusement, la dispersion colloïdale représente de 0,5 à 30 % en poids de la composition, mieux de 2 à 20 % et encore mieux de 2 à 15 %.

La composition mise en oeuvre dans l'invention peut comprendre, en outre, tout additif usuellement utilisé en cosmétique tels que les antioxydants, les conservateurs, les fibres, les parfums, les neutralisants, les épaississants, les vitamines, les hydratants, les filtres en particulier solaires, les agents de coalescence, les plastifiants, et leurs mélanges.

Bien entendu, l'homme du métier veillera à choisir les éventuels additifs complémentaires et/ou leur quantité de telle manière que les propriétés avantageuses de la composition ne soient pas, ou substantiellement pas, altérées par l'adjonction envisagée.

Les compositions peuvent être préparées selon des méthodes connues de l'homme du métier.

La composition utilisée dans le procédé selon l'invention peut être par exemple conditionnée dans un dispositif de conditionnement et d'application comprenant :
i) un support ;
ii) une surface d'application au moins en partie convexe disposée sur une face du support, la surface d'application étant formée par une partie au moins de la surface latérale d'un stick de ladite composition; et
iii) une pluralité d'éléments d'application, notamment sous forme de dents ou de poils, disposés selon au moins une rangée s'étendant d'un côté au moins de ladite surface d'application, et faisant saillie par rapport à ladite face du support.

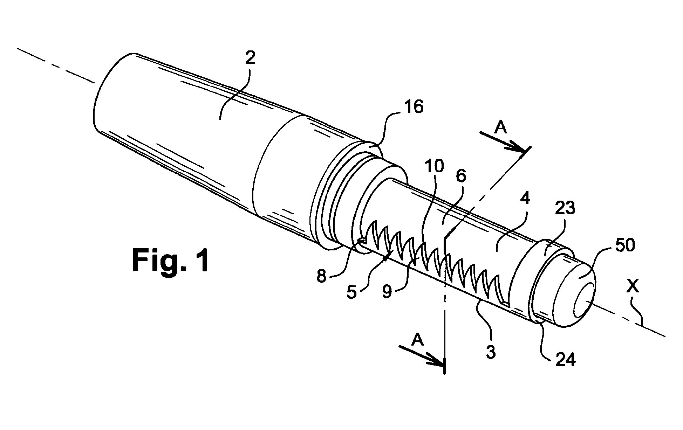

Un dispositif de conditionnement préféré est représenté à la figure 1 qui n'est présentée qu'à titre indicatif et nullement limitatif de l'invention. La figure 1 montre une vue de profil en perspective d'un mode de réalisation préférentiel d'un dispositif selon l'invention.
Le dispositif 1 comporte un organe de préhension 2 sur lequel est retenu un support 3 présentant l'organe d'application 4 s'étendant le long d'un axe longitudinal X.

Cet organe d'application 4 est cylindrique et est constitué par la composition sous forme de stick. L'organe d'application 4 comporte une surface d'application 6 apte à être mise en contact avec des fibres kératiniques sans qu'aucune portion du support 3 ne soit mise en contact avec lesdites fibres kératiniques.

L'organe d'application 4 repose sur une face du support 3, il y est par exemple retenu par des moyens de fixation tels qu'un anneau 23. Pour protéger la surface d'application 6 et l'organe d'application 4 entre deux utilisations, un capot de fermeture amovible (non représenté) est apte à être monté autour du support 3, et par exemple retenu sur ce dernier.

Le support 3 comporte une rangée 5 d'éléments d'application 9 s'étendant latéralement le long de l'organe d'application 4, de préférence contre le pourtour extérieur d'une partie de l'organe d'application 4. La surface d'application 6 est alors accessible au delà des extrémités libres 10 des éléments d'application 9 et également dans les espaces entre éléments d'application tels que 9.

Les exemples qui suivent sont présentés à titre illustratif de l'invention. Sauf indication contraire, les quantités sont données en gramme.

### Exemple 1 :

| | |
|---|---|
| Acetate isobutyrate de saccharose | 5 |
| (EASTMAN SAIB commercialisé par EASTMAN CHEMICAL) | |
| Mélange d'alcool gras à chaîne linéaire (C30-C50) et d'hydrocarbures C30-C50 (80/20) (Performacol 550L de NEW PHASE TECHNOLOGIES) | 2,5 |
| Cire de polyéthylène (POLYETHYLENE WAX AC 617 de Honeywell) | 10,5 |
| Dispersion de particules de poly(méthacrylate de méthyle/acide acrylique) stabilisées en surface dans de l'isododécane par un copolymère dibloc séquencé polystyrène/copoly(éthylènepropylène) vendu sous le nom de KRATON G1701, à 24,5% en matière Séche de polymères (Mexomère PAP de Chimex) | 67,82 |
| Oxyde de fer noir | 5 |
| Stéarate de l'oligomère de l'acide 12-polyhydroxystéarique | 0,16 |
| (Solsperse 21000 d'AVECIA) | |
| Isoparaffine (6-8 moles d'isobutylène) hydrogénée (PARLEAM de Nippon Oil Fats | 9 |

### Mode opératoire

On prépare une pâte pigmentaire de la façon suivante : on dissout le Solsperse 21000, dans le Parléam à environ 80°C pendant 10-15 min puis on ajoute l'oxyde de fer noir sous agitation au Rayneri pendant 15 min. L'ensemble est broyé à l'aide d'un broyeur à billes pendant environ 40 min.
Puis on fait fondre les cires avec l'acetate isobutyrate de saccharose, la pâte pigmentaire ci-dessus et le polyisobutène à 110°C dans un poëlon pendant environ 45 min sous agitation Rayneri.
Après homogénéisation du mélange, on ramène l'ensemble à 90°C puis on ajoute la dispersion de particules de polymère acrylate dans l'isododécane sous agitation. Après homogénéisation du mélange, le jus est coulé dans un moule alu siliconé à 42°C. On laisse reposer 10 min puis on racle la surface des sticks puis ceux ci sont placés pendant 45 min au congélateur à -28°C. Après recristallisation les sticks sont démoulés et introduits dans des conditionnements appropriés.

Ce mascara présente une dureté, mesurée selon le protocole indiqué plus haut, de 1814 Pa.

### Exemple 2 :

| | |
|---|---|
| Cire d'abeille | 15 |
| Cire de polyéthylène (PERFORMALENE 500 de NPT) | 0,5 |
| Polymethyl trifluoropropyl dimethylsiloxane (100 cSt) de Shin Etsu | 8,4 |
| Acetate isobutyrate de saccharose | 3 |
| (EASTMAN SAIB commercialisé par EASTMAN CHEMICAL) | |
| Mélange d'alcool gras à chaîne linéaire (C30-C50) et d'hydrocarbures | 2,5 |
| C30-C50 (80/20) (Performacol 550L de NEW PHASE TECHNOLOGIES) | |
| Phenyltrimethylsiloxy trisiloxane (20 cSt) (DC556 de Dow Corning) | 2,6 |
| Cyclopentadiméthylsiloxane (DC 245 Fluid de Dow Corning) | 58,5 |
| Stéarate de l'oligomère de l'acide 12-polyhydroxystéarique | 0,174 |
| (Solsperse 21000 d'AVECIA) | |
| Oxyde de fer noir | 5,22 |
| Isoparaffine (6-8 moles d'isobutylène) hydrogénée (PARLEAM de Nippon Oil Fat) | 7,1 |
| Isododécane | Qsp 100 |

### Mode opératoire

On prépare une pâte pigmentaire de la façon suivante : on dissout le Solsperse 21000, dans le Parléam à environ 80°C pendant 10-15 min puis on ajoute l'oxyde de fer noir sous agitation au Rayneri pendant 15 min. L'ensemble est broyé à l'aide d'un broyeur à billes pendant environ 40 min.
On fait fondre les cires avec l'acétate isobutyrate de saccharose, la pâte pigmentaire ci-dessus, le polyisobutène et la phenyltrimethylsiloxytrisiloxane à 110°C dans un poëlon pendant environ 45 min sous agitation Rayneri.
Après homogénéisation du mélange, on ramène l'ensemble à 90°C puis on ajoute la polymethyl trifluoropropyl dimethylsiloxane, la cyclopentadiméthylsiloxane et l'isododécane sous agitation Rayneri.
Après homogénéisation du mélange, le jus est coulé dans un moule alu siliconé à 42°C. On laisse reposer 10 min puis on racle la surface des sticks puis ceux ci sont placés pendant 45 min au congélateur à -28°C. Après recristallisation, les sticks sont démoulés et introduits dans des conditionnements appropriés.

Le mascara de l'exemple 2 présente une dureté, mesurée selon le protocole indiqué plus haut, de 2177 Pa.

## Revendications

1. Procédé de revêtement des cils comprenant l'application sur les dits cils d'au moins une couche d'au moins une composition présentée sous forme d'un stick, ladite composition étant apte à former un dépôt sans nécessiter de mise en contact préalable avec une phase aqueuse, la composition présentant une dureté allant de 900 à 10000 Pa conformément au protocole de mesure de la description, la composition comprenant une phase grasse liquide, la phase grasse liquide comprenant une huile volatile ou un mélange d'huiles volatiles, présentant dans la composition un profil d'évaporation, selon le protocole de la description, tel que la masse d'huile(s) volatile(s) évaporée(s) au bout de trente minutes va de 1,7 à 370 mg/cm², la composition comprenant au moins une cire non structurante et au moins une cire structurante, présentes en un ratio cire structurante / cire non structurante allant de 15/85 à 35/65, le caractère structurant ou non structurant de la cire étant défini à partir de la valeur de dureté obtenue sur un mélange binaire constitué de 15% cire et 85% de la ou des huiles de la phase grasse liquide de la composition conformément au protocole de mesure de la description étant considérée comme non structurante une cire dont le mélange binaire tel que défini ci-dessus présente une dureté inférieure à 35 g (± 2 g) étant considérée comme structurante une cire dont le mélange binaire tel que défini ci-dessus présente une dureté supérieure ou égale à 35 g (± 2 g).

2. Procédé selon la revendication 1, **caractérisé en ce que** la composition présente une dureté allant de 1800 à 8200 Pa.

3. Procédé selon la revendication 1 ou 2, **caractérisé en ce que** la phase grasse liquide comprend une huile volatile ou un mélange d'huiles volatiles, présentant dans la composition un profit d'évaporation tel que la masse d'huile(s) volatile(s) évaporée(s) au bout de trente minutes va de 2 à 70 mg/cm², et en particulier de 2 à 30 mg/cm².

4. Procédé selon l'une quelconque des revendications précédentes, **caractérisé en ce que** la phase grasse liquide de la composition comprend au moins une huile choisie parmi les huiles volatiles hydrocarbonées ayant de 8 à 16 atomes de carbone les huiles volatiles siliconées cycliques et leurs mélanges.

5. Procédé selon l'une quelconque des revendications précédentes, **caractérisé en ce que** la phase grasse liquide de la composition est présente en une teneur allant de 5 à 85 % en poids par rapport au poids total de la composition, de préférence de 10 à 70 % et de façon encore plus préférée de 15 à 60 % en poids.

6. Procédé selon l'une quelconque des revendications précédentes, **caractérisé en ce que** la cire structurante et/ou la cire non structurante sont choisies parmi les cires polaires, les cires apolaires et leurs mélanges.

7. Procédé selon l'une quelconque des revendications précédentes, **caractérisé en ce que** la ou les cire(s) structurante et non structurante représente(nt) de 1 à 30% en poids par rapport au poids total de la composition, de préférence de 5 à 20% et de façon encore plus préférée de 7,5 à 17% en poids

8. Procédé selon l'une quelconque des revendications précédentes, **caractérisé en ce que** la composition comprend au moins une phase grasse liquide comprenant au moins une huile hydrocarbonée volatile choisie parmi les isoparaffines ayant de 8 à 16 atomes de carbones, au moins une cire de polyéthylène et au moins une cire alcool gras.

9. Procédé selon l'une quelconque des revendications précédentes, **caractérisé en ce que** la composition comprend au moins une phase aqueuse.

10. Procédé selon l'une quelconque des revendications précédentes, **caractérisé en ce que** la composition comprend au moins un composé pâteux.

11. Procédé selon la revendication 10, **caractérisé en ce que** le composé pâteux représente de 0,5 à 85%, mieux 1 à 60%, mieux 2 à 30% et mieux encore 5 à 15% en poids de la composition.

12. Procédé selon l'une quelconque des revendications précédentes, **caractérisé en ce que** la composition comprend au moins un polymère filmogène.

13. Procédé selon la revendication 12. **caractérisé en ce que** le polymère filmogène est présent en une teneur en matières sèches allant de 0,1 % à 30 % en poids par rapport au poids total de la composition, de préférence de 0,5 % à 20 % en poids, et mieux de 1% à 15% en poids.

14. Procédé selon l'une des revendications précédentes, **caractérisé en ce que** la composition comprend une matière colorante.

15. Procédé selon la revendication 14, **caractérisé en ce que** la matière colorante représente de 0,01 à 30 % en poids par rapport au poids total de la composition.

## Claims

1. Process for coating eyelashes, comprising the application to the said eyelashes of at least one coat of at least one composition presented in the form of a stick, the said composition being capable of forming a deposit without the need for placing in contact beforehand with an aqueous phase, the composition having a hardness ranging from 900 to 10 000 Pa in accordance with the measuring protocol of the description, the composition comprising a liquid fatty phase, the liquid fatty phase comprising a volatile oil or a mixture of volatile oils, having in the composition an evaporation profile, according to the protocol of the description, such that the mass of volatile oil(s) evaporated after 30 minutes ranges from 1.7 to 370 mg/cm², the composition comprising at least one non-structuring wax and at least one structuring wax, with a structuring wax/non-structuring wax ratio ranging from 15/85 to 35/65, the structuring or non-structuring nature of the wax being defined from the hardness value obtained on a binary mixture formed from 15% wax and 85% oil(s) of the liquid fatty phase of the composition in accordance with the measuring protocol of the description, a non-structuring wax being considered as a wax whose binary mixture as defined above has a hardness of less than 35 g (± 2 g), and a structuring wax being considered as a wax whose binary mixture as defined above has a hardness of greater than or equal to 35 g (±2g).

2. Process according to Claim 1, **characterized in that** the composition has a hardness ranging from 1800 to 8200 Pa.

3. Process according to Claim 1 or 2, **characterized in that** the liquid fatty phase comprises a volatile oil or a mixture of volatile oils having in the composition an evaporation profile such that the mass of volatile oil(s) evaporated after thirty minutes ranges from 2 to 70 mg/cm² and in particular from 2 to 30 mg/cm².

4. Process according to any one of the preceding claims, **characterized in that** the liquid fatty phase of the composition comprises at least one oil chosen from hydrocarbon-based volatile oils containing from 8 to 16 carbon atoms and cyclic silicone volatile oils, and mixtures thereof.

5. Process according to any one of the preceding claims, **characterized in that** the liquid fatty phase of the composition is present in a content ranging from 5% to 85% by weight, preferably from 10% to 70% and even more preferably from 15% to 60% by weight relative to the total weight of the composition.

6. Process according to any one of the preceding claims, **characterized in that** the structuring wax and/or the non-structuring wax are chosen from polar waxes and apolar waxes, and mixtures thereof.

7. Process according to any one of the preceding claims, **characterized in that** the structuring and non-structuring wax(es) represent(s) from 1% to 30% by weight, preferably from 5% to 20% and even more preferably from 7.5% to 17% by weight relative to the total weight of the composition.

8. Process according to any one of the preceding claims, **characterized in that** the composition comprises at least one liquid fatty phase comprising at least one volatile hydrocarbon-based oil chosen from isoparaffins containing from 8 to 16 carbon atoms, at least one polyethylene wax and at least one fatty alcohol wax.

9. Process according to any one of the preceding claims, **characterized in that** the composition comprises at least one aqueous phase.

10. Process according to any one of the preceding claims, **characterized in that** the composition comprises at least one pasty compound.

11. Process according to Claim 10, **characterized in that** the pasty compound represents from 0.5% to 85%, better still from 1% to 60%, better still from 2% to 30% and even better still from 5% to 15% by weight of the composition.

12. Process according to any one of the preceding claims, **characterized in that** the composition comprises at least one film-forming polymer.

13. Process according to Claim 12, **characterized in that** the film-forming polymer is present in a solids content ranging from 0.1% to 30% by weight, preferably from 0.5% to 20% by weight and better still from 1% to 15% by weight relative to the total weight of the composition.

14. Process according to one of the preceding claims, **characterized in that** the composition comprises a dyestuff.

15. Process according to Claim 14, **characterized in that** the dyestuff represents from 0.01% to 30% by weight relative to the total weight of the composition.

## Patentansprüche

1. Verfahren zur Beschichtung der Wimpern, welches das Aufbringen mindestens einer Schicht aus mindestens einer Zusammensetzung, die in Form eines Stifts vorliegt, auf die Wimpern umfasst, wobei die Zusammensetzung dazu geeignet ist, eine Ablagerung zu bilden, ohne dass im Vorfeld ein Kontakt mit einer wässrigen Phase erforderlich wäre, wobei die Zusammensetzung eine Härte aufweist, die gemäß der Messanleitung in der Beschreibung im Bereich von 900 bis 10.000 Pa liegt, wobei die Zusammensetzung eine flüssige Fettphase umfasst, wobei die flüssige Fettphase ein flüchtiges Öl oder eine Mischung flüchtiger Öle umfasst, das/die in der Zusammensetzung ein Verdampfungsprofil gemäß der Anleitung in der Beschreibung aufweist, das derart beschaffen ist, dass die Masse an flüchtigen/flüchtigem Öl (en), die nach dreißig Minuten verdampft ist, 1,7 bis 370 mg/cm² beträgt, wobei die Zusammensetzung mindestens ein nicht-strukturierendes Wachs und mindestens ein strukturierendes Wachs umfasst, welche in einem Verhältnis von strukturierendem Wachs / nicht-strukturierendem Wachs vorliegen, das 15/85 bis 35/65 beträgt, wobei die strukturierende oder nicht-strukturierende Beschaffenheit des Wachses anhand des Härtewertes bestimmt wird, der gemäß der Messanleitung in der Beschreibung bei einer binären Mischung ersielt wird, die aus 15 % Wachs und 85 % des Öls oder der Öle der flüssigen Fettphase der Zusammensetzung besteht, wobei ein wachs, dessen binäre Mischung gemäß der obigen Begriffsbestimmung eine Härte von weniger als 35 g (± 2 g) aufweist, als nichtstrukturierend angesehen wird und ein Wachs, dessen binäre Mischung gemäß der obigen Begriffsbestimmung eine Härte größer gleich 35 g (± 2 g) aufweist, als strukturierend angesehen wird.

2. Verfahren nach Anspruch 1, **dadurch gekennzeichnet, dass** die Zusammensetzung eine Härte aufweist, die im Bereich von 1.800 bis 8.200 Pa liegt.

3. Verfahren nach Anspruch 1 oder 2, **dadurch gekennzeichnet, dass** die flüssige Fettphase ein flüchtiges Öl oder ein Gemisch flüchtiger Öle umfasst, das/die in der Zusammensetzung ein Verdampfungsprofil aufweisen, das derart beschaffen ist, dass die Masse an flüchtigen/flüchtigem Öl (en), die nach dreißig Minuten verdampft ist, 2 bis 70 mg/cm² und inbesondere 2 bis 30 mg/cm² beträgt.

4. Verfahren nach einem beliebigen der vorhergehenden Ansprüche, **dadurch gekennzeichnet, dass** die flüssige Fettphase der Zusammensetzung mindestens ein Öl umfasst, das aus den flüchtigen Ölen aus Kohlenwasserstoffen mit 8 bis 16 Kohlenstoffatomen, den flüchtigen Ölen aus cyclischen Silikonen und deren Mischungen ausgewählt ist.

5. Verfahren nach einem beliebigen der vorhergehenden Ansprüche, **dadurch gekennzeichnet, dass** die flüssige Fettphase der Zusammensetzung in einem Gehalt vorliegt, der bei 5 bis 85 Gew.-% liegt, bezogen auf das Gesamtgewicht der Zusammensetzung, vorzugsweise bei 10 bis 70 % und mit noch größerem Vorzug bei 15 bis 60 Gew.-%.

6. Verfahren nach einem beliebigen der vorhergehenden Ansprüche, **dadurch gekennzeichnet, dass** das strukturierend Wachs und/oder das nicht-strukturierende Wachs aus den polaren Wachsen, den unpolaren Wachsen und deren Mischungen ausgewählt ist.

7. Verfahren nach einem beliebigen der vorhergehenden Ansprüche, **dadurch gekennzeichnet, dass** das oder die strukturierende(n) und nicht-strukturierende(n) Wachs(e) 1 bis 30 Gew.-% ausmachen, bezogen auf das Gesamtgewicht der Zusammensetzung, vorzugsweise 5 bis 20 % und mit noch größerem Vorzug 7,5 bis 17 Gew.-%.

8. Verfahren nach einem beliebigen der vorhergehenden Ansprüche, **dadurch gekennzeichnet, dass** die Zusammensetzung mindestens eine flüssige Fettphase umfasst, die mindestens ein flüchtiges Kohlenwasserstoff-Öl, das aus den Isoparaffinen mit 8 bis 16 Kohlenstoffatomen ausgewählt ist, mindestens ein Polyethylenwachs und mindestens ein Fettalkoholwachs umfasst.

9. Verfahren nach einem beliebigen der vorhergehenden Ansprüche, **dadurch gekennzeichnet, dass** die Zusammensetzung mindestens eine wässrige Phase umfasst.

10. Verfahren nach einem beliebigen der vorhergehenden Ansprüche, **dadurch gekennzeichnet, dass** die Zusammensetzung mindestens eine pastöse Verbindung umfasst.

11. Verfahren nach Anspruch 10, **dadurch gekennzeichnet, dass** die pastöse Verbindung 0,5 bis 85%, besser 1 bis 60 %, besser 2 bis 30 % und noch besser 5 bis 15 Gew.-% der Zusammensetzung ausmacht.

12. Verfahren nach einem beliebigen der vorhergehenden Ansprüche, **dadurch gekennzeichnet, dass** die Zusammensetzung mindestens ein filmbildendes Polymer umfasst.

13. Verfahren nach Anspruch 12, **dadurch gekennzeichnet, dass** das filmbildende Polymer zu einem Trockenmassegehalt von 0,1 bis 30 Gew.-% vorliegt, bezogen auf das Gesamtgewicht der Zusammensetzung, vorzugsweise von 0,5 bis 20 Gew.-%, und besser von 1 bis 15 Gew.-%.

14. Verfahren nach einem der vorhergehenden Ansprüche, **dadurch gekennzeichnet, dass** die Zusammensetzung einen Farbstoff enthält.

15. Verfahren nach Anspruch 14, **dadurch gekennzeichnet, dass** der Farbstoff 0,01 bis 30 Gew.-% ausmacht, bezogen auf das Gesamtgewicht der Zusammensetzung.
